# EUROPÄISCHE PATENTSCHRIFT

(11) **EP 0 956 879 B1**
(45) Veröffentlichungstag und Bekanntmachung des Hinweises auf die Patenterteilung: **07.07.2004**
(21) Anmeldenummer: 99810409.5
(22) Anmeldetag: 07.05.1999
(51) Int. Cl.: A61M 25/00, A61M 25/06, A61M 39/02

(54) **Infusionsvorrichtung für subkutane Verabreichung eines Wirkstoffs**
Subcutaneous drug delivery device
Dispositif de perfusion pour délivrer un agent de manière sous-cutané

(30) Priorität: 14.05.1998 DE 19821723
(43) Veröffentlichungstag der Anmeldung: 17.11.1999
(73) Patentinhaber: Disetronic Licensing AG, 3401 Burgdorf (CH)
(72) Erfinder: Marggi, Rolf, 3006 Bern (CH)
(74) Vertreter: Schwabe - Sandmair - Marx

(56) Entgegenhaltungen:
- EP-A- 0 792 658
- US-A- 5 522 803

## Beschreibung

Die Erfindung betrifft einen Katheterkopf für subkutane Verabreichung eines Wirkstoffs, insbesondere eines medizinischen Wirkstoffs wie beispielsweise Insulin. Der Katheterkopf umfaßt ein Kanülengehäuse mit einer Kanüle und einen mit dem Kanülengehäuse zu verbindenden Nadelhalter mit einer Zuführung für den Wirkstoff. Die Kanüle ragt von dem Kanülengehäuse ab und wird in einem Gewebe plaziert. Die Kanüle kann einstückig mit dem Kanülengehäuse ausgebildet sein, in einer bevorzugten anderen Ausführungsform ist sie im Kanülengehäuse befestigt bzw. verankert. Sie kann starr, beispielsweise als Stahlkanüle, oder nachgiebig, insbesondere biegbar, ausgebildet sein. In dem Kanülengehäuse ist für den Wirkstoff ein Durchgangskanal zur Kanüle ausgebildet. Das Kanülengehäuse ist so geformt, daß es auf dem Gewebe, in dem die Kanüle plaziert ist, flächig aufsitzt, und es ist für eine Fixierung bzw. Befestigung auf dem Gewebe vorbereitet.

Am Nadelhalter ist eine Verbindungsnadel starr befestigt, die zum Herstellen der Verbindung in den Durchgangskanal des Kanülengehäuses eingeführt wird. Die Kanüle und das Kanülengehäuse verbleiben am Ort der Fixierung in und auf dem Gewebe, während der Nadelhalter wiederholt mit dem Kanülengehäuse verbunden und wieder davon gelöst werden kann. Vorzugsweise verrasten sie bei dem Zusammenfügen miteinander automatisch, insbesondere indem sich der Nadelhalter lösbar am Kanülengehäuse verankert. Zur Positionierung der Verbindungsnadel relativ zum Durchgangskanal des Kanülengehäuses und zum Einführen der Verbindungsnadel in den Durchgangskanal sind Führungsmittel vorgesehen, die den Nadelhalter an dem Kanülengehäuse führen.

Ein Katheterkopf der vorgenannten Art ist aus der US-PS 5,522,803 bekannt. Die Führungsmittel des bekannten Katheterkopfes werden durch ein Paar von Führungsstiften gebildet, die beidseits der Verbindungsnadel sowie parallel dazu vom Nadelhalter abragen. Das Kanülengehäuse ist entsprechend beidseits eines Einlasses in den Durchgangskanal mit Führungsschächten versehen, in die je einer der Führungsstifte zum Einführen der Verbindungsnadel einfährt. Die Verbindungsnadel wird durch das Zusammenwirken dieser Führungsstifte mit den Führungsschächten relativ zum Einlaß positioniert und beim Einführen in den Durchgangskanal zentriert geführt. Im Verlaufe des Vorschiebens der Verbindungsnadel im Durchgangskanal wird der Nadelhalter durch eine selbsttätig verrastende Schnappverbindung am Kanülengehäuse verankert.

Die Erfindung hat es sich zur Aufgabe gemacht, einen Katheterkopf für eine subkutane Wirkstoffverabreichung zu schaffen, dessen Kanülengehäuse und Nadelhalter auf einfache Weise korrekt miteinander verbunden werden können und der einfach herstellbar ist.

Nach der Erfindung wird bei einem Katheterkopf der eingangs beschriebenen Art ein am Nadelhalter ausgebildetes Führungsmittel durch eine die Verbindungsnadel axial umgebende Führungshülse gebildet, die bei einem Zusammenfügen des Nadelhalters und des Kanülengehäuses, d. h., bei dem Einführen der Verbindungsnadel eng gleitgeführt über einen zylindrischen Fortsatz des Kanülengehäuses geschoben wird. Der zylindrische Fortsatz umschließt den Einlaß und einen sich daran anschließenden Bereich des Durchgangskanals des Kanülengehäuses. Die Erfindung stellt ein sicheres Einführen der Verbindungsnadel sicher, ohne die zusätzliche Ausbildung von Führungsstiften zu erfordern. Ferner stellt die Führungshülse einen Handhabungsschutz sowohl für die Nadel als auch den Verwender dar. Die Führungshülse kann durchbrochen sein, vorzugsweise ist sie jedoch als geschlossener Hülsenkörper ausgebildet.

In einem bevorzugten Ausführungsbeispiel weist das Kanülengehäuse einen kompakten vorderen Bereich auf, von dessen Unterseite die Kanüle abragt und von dessen Rückseite übereinanderliegend ein scheibenförmiger Bereich, der die auf dem Gewebe aufliegende Unterseite des Kanülengehäuses verlängert, und der zylindrische Fortsatz abragen. Eine dem zylindrischen Fortsatz zugewandte Oberseite des scheibenförmigen hinteren Bereichs des Kanülengehäuses und eine Unterseite des Nadelhalters wirken beim Zusammenfügen des Kanülengehäuses und des Nadelhalters als weitere Führungsmittel, die ein Drehen des Nadelhalters relativ zum Kanülengehäuse um die Längsachse der Verbindungsnadel verhindern.

Bevorzugterweise ist in dem Kanülengehäuse ein zusätzlicher Durchgang für eine Einstichnadel für die Kanüle ausgebildet. Dieser Durchgang weist im zusammengefügten Zustand von Kanülengehäuse und Nadelhalter winklig zur Verbindungsnadel. Der die Verbindungsnadel aufnehmende Durchgangskanal mündet in diesen zusätzlichen Durchgang ein, der nach dem Plazieren der Kanüle und Herausziehen der Einstichnadel den Durchgangskanal bis zur Kanüle verlängert. Indem eine Einstichnadel nicht aus dem Durchgangskanal herausgezogen werden muß, in den nach dem Plazieren der Kanüle und Befestigen des Kanülengehäuses die Verbindungsnadel eingeführt wird, ist ein komplettes Vorfüllen des Katheterkopfes bis zur Kanüle, d.h. ein Primen, im zusammengefügten Zustand von Kanülengehäuse und Nadelhalter möglich.
In einer weiteren Ausführungsform verlängert eine nachgiebige Kanüle den Durchgangskanal jedoch fluchtend. In einer dritten Ausführungsform wird die Kanüle durch die Einstichnadel selbst gebildet.

In einem bevorzugten Herstellungsverfahren wird die vorgefertigte Kanüle bei einem Spritzgießen des Kanülengehäuses in das Kanülengehäuse gleich eingespritzt. Hierfür weist die Kanüle in einem rückwärtigen Bereich vorzugsweise eine Verbreiterung auf, die beim Einspritzen die Kanüle im Kanülengehäuse verankert. Die Kanüle wird vorzugsweise aus einem weichen Kunststoffmaterial, insbesondere Teflon, hergestellt. Als Material für das Kanülengehäuse und auch für den Nadelhalter kann ein thermoplastischer Kunststoff verwendet werden.

Bevorzugte Ausführungsbeispiele der Erfindung werden nachfolgend anhand von Figuren erläutert. Es zeigen:
- Figur 1: einen Katheterkopf im zusammengefügten Zustand,
- Figur 2: den Katheterkopf nach Figur 1, wobei Komponenten des Katheterkopfes aus ihren Einbaulagen herausgelöst dargestellt sind,
- Figur 3: den Katheterkopf nach Figur 2 unmittelbar vor dem Zusammenfügen,
- Figur 4: einen Längsschnitt des Katheterkopfs nach den Figuren 1 bis 3 im zusammengefügten Zustand,
- Figur 5: ein zweites Ausführungsbeispiel eines Katheterkopfs, und
- Figur 6: den Katheterkopf nach Figur 5 in einem Längsschnitt.

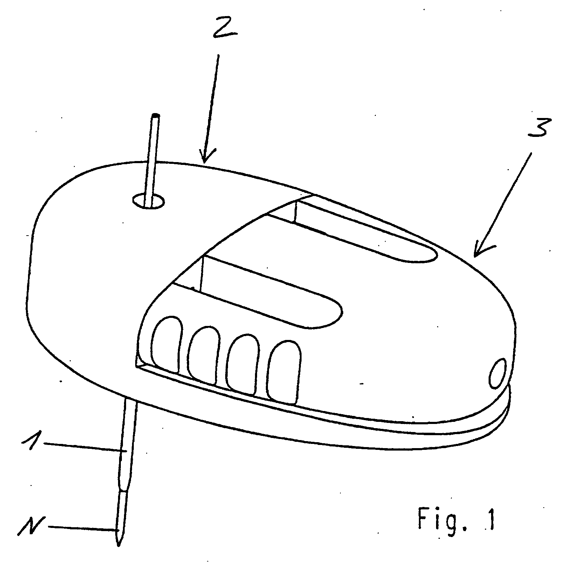

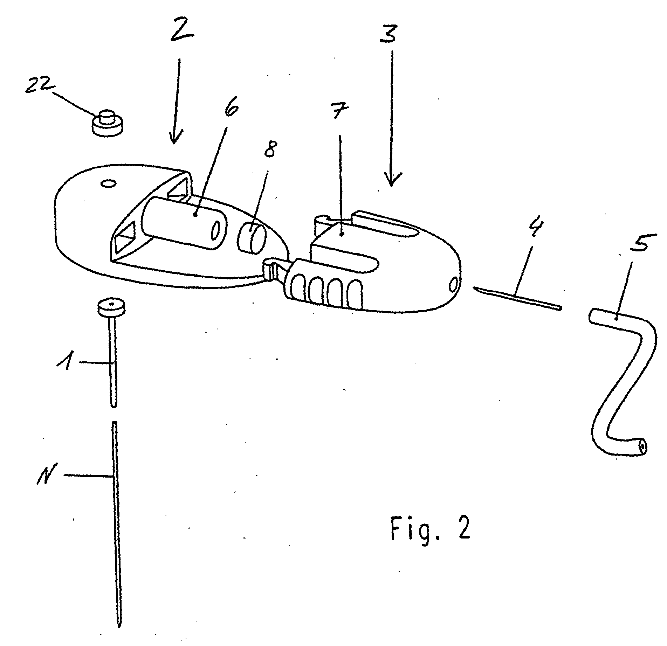

Figur 1 zeigt einen Katheterkopf mit einer senkrecht von einer Unterseite des Katheterkopfs abragenden Kanüle 1. Die Kanüle 1 aus einem weichen Kunststoffmaterial, im Ausführungsbeispiel Teflon, umschließt eine Einstichnadel N eng, die den Katheterkopf senkrecht zu dessen planen Unterseite durchragt. Der Katheterkopf der Figur 1 bildet das vordere Ende eines Katheters 5, der in den Figuren 2 bis 4 angedeutet ist. Der Katheter mit dem Katheterkopf wird von einem Verwender, beispielsweise einem Diabetiker, selbst gesetzt. Hierbei werden die Einstichnadel N und die Kanüle 1 senkrecht unter die Haut in das Gewebe gestochen und der Katheterkopf mit seiner Unterseite flächig aufliegend auf der Haut fixiert bzw. befestigt. Die Fixierung erfolgt mittels einer selbsthaftenden Rondelle bzw. einem Pflaster. Solch eine Rondelle vergrößert die für Haftzwecke zur Verfügung stehende Unterseite des Katheterkopfs. Falls diese Unterseite selbst bereits eine ausreichend große Haftfläche bereitstellt, genügt bereits die Ausbildung dieser Unterseite als Haftfläche. Die Einstichnadel N wird nach dem Plazieren der Kanüle 1 aus dem Katheterkopf herausgezogen, so daß im Gewebe lediglich noch die dünne, nachgiebige, insbesondere biegbare, Kanüle 1 verbleibt.

Der Katheterkopf weist ein Kanülengehäuse 2 auf, das zusammen mit der Kanüle 1 an der Einstichstelle verbleibt, insbesondere also die zur Fixierung des Katheterkopfs dienende Unterseite aufweist, und einen Nadelhalter 3, der das vordere Ende des Katheters 5 bildet. Das Kanülengehäuse 2 und der Nadelhalter 3 gehen miteinander eine wiederholt herstellbare und wieder lösbare Steckverbindung ein.

In Figur 2 sind das Kanülengehäuse 2 und der Nadelhalter 3 voneinander getrennt, jedoch bereits zum Zusammenstecken relativ zueinander ausgerichtet dargestellt. Ferner sind die in separaten Herstellungsschritten zu fertigenden Einzelkomponenten des Katheterkopfs aus ihren Einbaulagen herausgelöst einzeln dargestellt. Sämtliche Einzelkomponenten sind relativ zueinander entsprechend ihren jeweiligen Einbaulagen ausgerichtet.

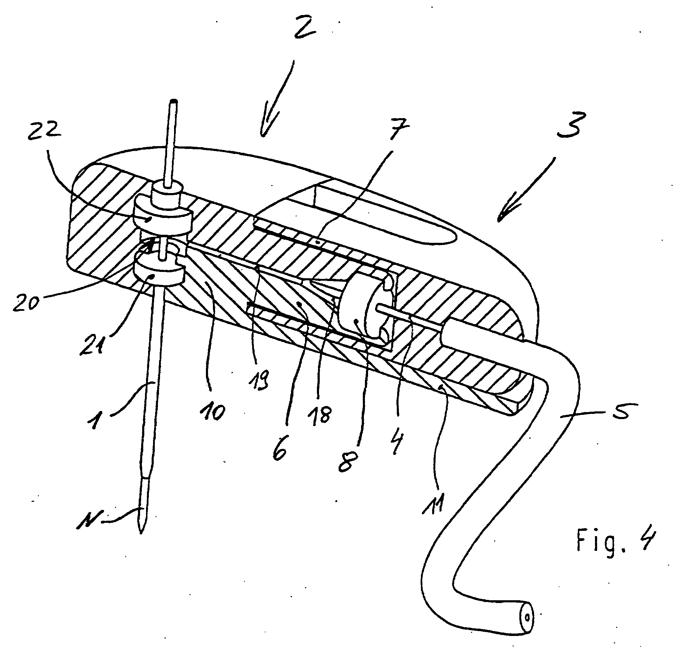

Der Wirkstoff wird durch den Katheter 5 zum Nadelhalter 3 herangeführt. Er wird durch eine im Nadelhalter 3 aufgenommene Verbindungsnadel 4 hindurch in einen Durchgangskanal im Kanülengehäuse 2 geleitet, darin zur Kanüle 1 weitertransportiert und gelangt durch die Kanüle 1 hindurch an den gewünschten Ort im Gewebe. Die in den Figuren 1 bis 4 noch dargestellte Einstichnadel N ist hierbei bereits entfernt worden. Ein Einlaß 9 und ein sich daran anschließender Bereich des Durchgangskanals des Kanülengehäuses 2 werden von einem zylindrischen Fortsatz 6 umschlossen, der von einer Rückseite des Kanülengehäuses 2 abragt. Der Transportweg im Katheterkopf ist im Detail am besten im Längsschnitt der Figur 4 zu erkennen.

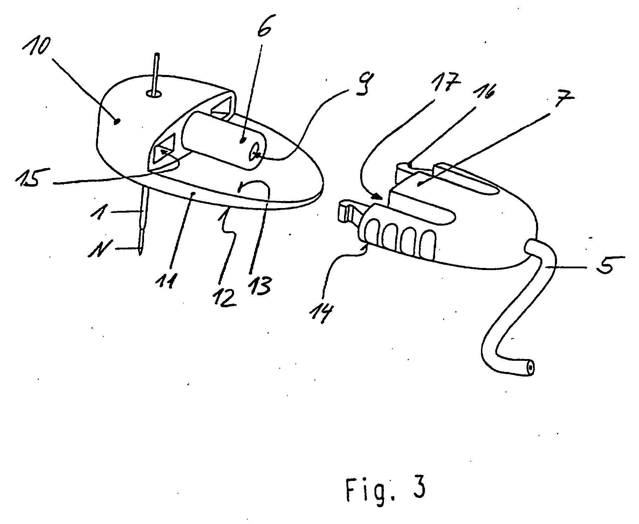

Figur 3 zeigt das Kanülengehäuse 2 und den Nadelhalter 3 jeweils komplettiert ebenfalls in der für das Zusammenstecken geeigneten Stellung relativ zueinander. Aus dieser Stellung wird der Nadelhalter 3 in Längsrichtung der auf den Einlaß 9 weisenden Verbindungsnadel 4 auf das Kanülengehäuse 2 geradlinig zugeführt und aufgesteckt. Die Zuführ- und Aufsteckrichtung des Nadelhalters 3 weist im wesentlichen parallel zur Hautoberfläche. Der Katheder wird somit parallel zur Hautoberfläche weggeführt; er kann auch zur Hautoberfläche hin geneigt weggeführt werden.

Der Katheterkopf hat im zusammengefügten Zustand insgesamt die Form eines Halbovaloids mit einer planen Unterseite 12, die zum Rand hin von der Haut abfallend gerundet ausläuft und übergeht in eine darüber bereichsweise konvex und bereichsweise konkav sich wölbende Oberseite. Das Kanülengehäuse 2, an dem die auf der Haut liegende Unterseite 12 ausgebildet ist, weist einen hinteren scheibenförmigen Bereich 11 und einen demgegenüber verdickten vorderen Bereich 10 auf, von dessen Unterseite 12 die Kanüle 1 und von dessen Rückseite der zylindrische Fortsatz 6 und diesen überdeckend der scheibenförmige Bereich 11 nach hinten in Richtung auf den dagegen zuzuführenden Nadelhalter 3 abragen. Die Oberseite 13 des hinteren Bereichs 11 ist konkav gerundet in Anpassung an die entsprechend nach außen sich wölbende Unterseite 14 des Nadelhalters 13. Die Oberseite des vorderen Bereichs 10 ist demgegenüber nach außen konvex vorgewölbt. Der Nadelhalter 3 weist eine symmetrische Form auf, d.h. seine Oberseite und seine Unterseite 14 sind in gleicher Weise nach außen gewölbt; ferner ist der Nadelhalter 3 symmetrisch in der Draufsicht bezüglich seiner Mittellängsachse. Die Unter- und Oberseite des Nadelhalters 3 können aufgrund der Symmetrie beim Zusammenstecken vertauscht werden. Die Handhabung wird hierdurch erleichtert, da auch allein durch Tasten die korrekte Ausrichtung ohne weiteres verifiziert werden kann.

Der zylindrische Fortsatz 6 dient im Zusammenwirken mit einer an dem Nadelhalter 3 ausgebildeten Führungshülse 7 als Führungsmittel zum Positionieren der Verbindungsnadel 4 relativ zum Einlaß 9 und zur korrekten Geradführung der Verbindungsnadel 4 im sich an den Einlaß 9 anschließenden Bereich des Durchgangskanals. Erfindungsgemäß wird somit ein den Durchgangskanal umschließender Teil des Kanülengehäuses 2 in Form des zylindrischen Fortsatzes 6 aus dem Kanülengehäuse 2 herausgeführt und kann so als Führungsmittel beim Einführen der Verbindungsnadel 4 genutzt werden. Das damit zusammenwirkende Führungsmittel am Nadelhalter 3 wird durch die Führungshülse 7 gebildet, die über ihre Funktion als Führungsmittel hinaus gleichzeitig die in ihr koaxial aufgenommene Verbindungsnadel 4 vor Beschädigungen schützt; zusätzlich schützt sie einen Verwender vor etwaigen Verletzungen aufgrund einer vorstehenden Nadel, beispielsweise bei einem Ertasten oder generell bei unachtsamer Handhabung. Die Verbindungsnadel 4 wird in ihrer Längsrichtung von der Führungshülse 7 überragt.

Die Führungshülse 7 wird durch Ausnehmen von zwei Längsschlitzen 17 gebildet, d.h. sie bleibt zwischen diesen beiden Schlitzen 17 als hülsenartiger Fortsatz stehen. Grundsätzlich könnte die Führungshülse 7 durch den Nadelhalter 3 insgesamt gebildet werden, d.h. sie wäre dann als geradzylindrische Ausnehmung im ansonsten vollen Nadelhalter 3 gebildet, der dann insgesamt als Führungshülse zu bezeichnen wäre.

Durch die Schlitze 17 werden jedoch beidseits der Führungshülse 7 zwei elastische Schnapperfinger 16 gebildet, die über die Führungshülse 7 hinaus vorragen. Beim Aufstecken des Nadelhalters 3 greifen die Schnapperfinger 16 in entsprechende Führungsschächte 15 ein, die beidseits des zylindrischen Fortsatzes 6 im Kanülengehäuse 2 vorgesehen sind. Während des Einführens der Verbindungsnadel 4 gleiten die Schnapperfinger 16 an konisch aufeinander zulaufenden Führungsflächen ihrer Führungsschächte 15 entlang und werden hierdurch aufeinander zu gebogen. Hinter Vorsprüngen, die in den Führungsschächten 15 ausgebildet sind, schnappen die elastisch biegbaren Schnapperfinger 16 mit ihren Rastnasen nach außen, wenn die Verbindungsnadel 4 komplett eingeführt worden ist, und verankern derart die entsprechenden Vorsprünge der Führungsschächte hintergreifend den Nadelhalter 3 am Kanülengehäuse 2. Die Schnappverbindung wird gelöst, indem die Schnapperfinger 16 innerhalb ihrer geriffelten Bereiche aufeinander zu gedrückt werden. Nachdem auf diese Weise der Hintergriff gelöst worden ist, kann der Nadelhalter 3 vom Kanülengehäuse 2 abgezogen werden.

Figur 4 zeigt den Katheterkopf im Längsschnitt im zusammengefügten Zustand. Die Führungshülse 7 ist vollkommen auf den zylindrischen Fortsatz 6 aufgeschoben und stößt mit ihrer vorderen Kante gegen die Rückseite des Kanülengehäuses 2, von der der zylindrische Fortsatz 6 abragt. In diesem Zustand hintergreifen die Schnapperfinger 16 die entsprechenden Vorsprünge in den Führungsschächten 15. Ein unbeabsichtigtes Lösen des Nadelhalters 3 ist nicht möglich.

Bei dem Aufstecken des Nadelhalters 3 gleitet der Nadelhalter 3 mit seiner Unterseite 14 auf der angeschmiegt gewölbten Oberseite 13 des Kanülengehäuses 2. Zum Positionieren der Verbindungsnadel 4 wird die Führungshülse 7 in Flucht zum zylindrischen Fortsatz 6 gebracht, wobei der Nadelhalter 3 auf der Oberseite 13 des Kanülengehäuses 2 gestützt verschoben werden kann. Durch leichtes Abrunden des vorderen Randes des zylindrischen Fortsatzes 6 wird der Fortsatz 6 zu Beginn des Überschiebens in der Führungshülse 7 zentriert. Anschließend wird der Nadelhalter 3 mit seiner Führungshülse 7 über den zylindrischen Fortsatz 6 vorgeschoben. Dabei durchsticht die Verbindungsnadel 4 ein unmittelbar hinter dem Einlaß 9 im Durchgangskanal des zylindrischen Fortsatzes 6 angeordnetes Septum 8. Das Septum 8 ist so ausgebildet, daß es auch nach mehrfachem Durchstechen den Durchgangskanal des Kanülengehäuses 2 hermetisch verschließt. Unmittelbar hinter dem Septum 8 weist der Durchgangskanal einen Dom 18 auf, in den die Verbindungsnadel 4 hineinragt. An den Dom 18 schließt sich ein in Flucht zur Verbindungsnadel 4 geradlinig verlaufender Kanalabschnitt 19 an, der in einen Hohlraum 20 im vorderen Bereich 10 des Kanülengehäuses 2 einmündet. Auch die Kanüle 1 mündet in diesen Hohlraum 20 ein. Durch den Hohlraum 20 ist die Einstichnadel N winkelig, im Ausführungsbeispiel rechtwinklig, zur Verbindungsnadel 4 und dem Kanalabschnitt 19 hindurchgeführt. Die Einstichnadel N durchragt das Kanülengehäuse 2 und weist winklig, im Ausführungsbeispiel rechtwinklig, zu dessen Unterseite 12. In dieser Anordnung wird die Einstichnadel N vorteilhafterweise nicht durch denjenigen Teil des Durchgangskanals des Kanülengehäuses 2 geführt, in den die Verbindungsnadel 4 eingeführt wird. Durch diese Anordnung muß nicht erst die Einstichnadel N entfernt werden, um die Verbindungsnadel in das Kanülengehäuse einführen zu können. Dies ist insbesondere für das sogenannte Primen von Vorteil, bei dem der Katheterkopf vor dem Plazieren der Kanüle 1 möglichst vollständig mit dem Wirkstoff gefüllt wird. Dies erleichtert die Handhabung erheblich.

Die Kanüle 1 ist als dünner Schlauch ausgebildet mit einer flanschartigen Verbreiterung 21 an einem Ende. Die flanschartige Verbreiterung 21 wird in einer ringscheibenförmigen Ausnehmung im Kanülengehäuse 2 aufgenommen und die Kanüle 1 dadurch verankert.

Dem Kanüleneinlaß gegenüber ist im Hohlraum 20 ein weiteres Septum 22 eingelassen, das den Hohlraum 20, der Teil des Durchgangskanals des Kanülengehäuses 2 ist, nach dem Herausziehen der Einstichnadel N abdichtet. Die Wirkungsweise des Septums 22 ist dem des Septums 8 vergleichbar. Die Form des Hohlraums 20 ist im wesentlichen zylindrisch, wobei die flanschartige Verbreiterung 21 der Kanüle 1 und das Septum 22 die einander gegenüberliegenden Stirnflächen des zylindrischen Hohlraums 20 bilden und zwischen denen der Kanalabschnitt 19 einmündet. Zum Zwecke des Primens weist die Einstichnadel N in ihrem zwischen der flanschartigen Verbreiterung 21 und dem Septum 22 liegenden Abschnitt eine Öffnung auf.

In Figur 4 ist die vollflächig plane Auflage des Nadelhalters 3 auf der Oberseite 13 des scheibenförmigen hinteren Bereichs 11 des Kanülengehäuses 2 deutlich erkennbar. Erkennbar ist ferner, daß zwischen der Oberseite 13 und dem zylindrischen Fortsatz 6 ein lichter Abstand verbleibt, in den die Führungshülse 7 bei dem Aufstecken des Nadelhalters 3 einfährt. Grundsätzlich ist solch eine Beabstandung zwischen dem scheibenförmigen Bereich 11 und dem zylindrischen Fortsatz 6 nicht erforderlich. Der zylindrische Fortsatz 6 könnte beispielsweise flach unmittelbar auf dem scheibenförmigen Bereich 11 aufsitzen. In dieser Ausbildung wäre die Führungshülse 7 an ihrer Unterseite entsprechend offen. Die innere Mantelfläche als eigentliche Führung der Führungshülse 7 und die äußere Mantelfläche des zylindrischen Fortsatzes 6 müssen auch nicht kreiszylindrisch geformt sein, obgleich dies bevorzugt wird. Sie können beispielsweise auch durch Rechteckzylinder gebildet werden. Wesentlich ist jedoch, daß ausreichend geradzylindrische Führungsflächen zum Positionieren und sauberen Führen der Verbindungsnadel 4 vorhanden sind. Die Führung und insbesondere die Verdrehsicherheit des Nadelhalters 3 relativ zum fixierten Kanülengehäuse 2 wird durch die als Führungsflächen dienende Oberseite 13 und Unterseite 14 des Kanülengehäuses 2 bzw. des Nadelhalters 3 noch verbessert. Die Form dieser beiden Führungsflächen 13 und 14 sorgt bereits für die korrekte Ausrichtung, insbesondere Zentrierung, beim Zusammenfügen.

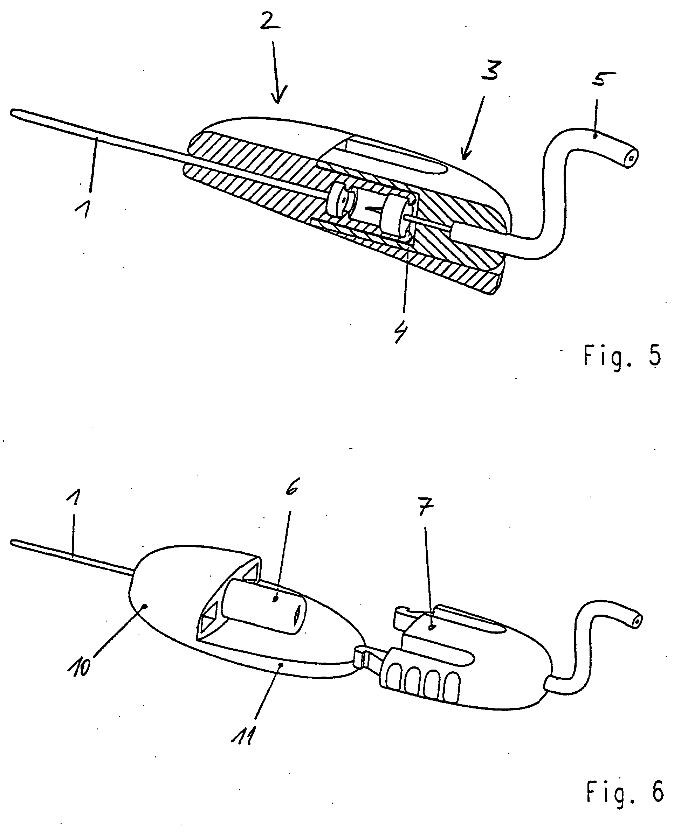

Die Figuren 5 und 6 zeigen ein abgewandeltes Ausführungsbeispiel, bei dem die Einstichnadel durch den gleichen Durchgangskanal des Kanülengehäuses 2 gestochen wird, in den nach dem Plazieren der Kanüle 1 die Verbindungsnadel 4 einzuführen ist. Mit Ausnahme dieser Anordnung der Einstichnadel und der Kanüle 1 entspricht der Katheterkopf der Figuren 5 und 6 dem vorbeschriebenen, so daß auf dessen Beschreibung verwiesen wird.

Das Kanülengehäuse 2 wird in beiden Ausführungsbeispielen nach der Erfindung in einem einzigen Spritzgießschritt fertiggestellt. Hierfür wird die vorgefertigte Kanüle 1 mit ihrer Verbreiterung 21, gegebenenfalls auch das Septum 8 und das Septum 22, in die Spritzgießform eingelegt und beim Spritzgießen des Kanülengehäuses 2 unmittelbar darin eingespritzt. Die erforderlichen Fixierungen in Form von die vorgenannten Teile einfassenden Umlaufschultern werden durch die Spritzform vorgegeben. Die Septen 8 und 22 können auch in das Kanülengehäuse 2 eingelegt werden, wobei dann in einem weiteren Verfahrensschritt das Kanülengehäuse 2 zum Halten der Septen 8 und 22 umgeformt wird. Insbesondere das Einspritzen der Kanüle 1 stellt eine erhebliche Verfahrenserleichterung bei der Herstellung des Kanülengehäuses 2 dar.

## Patentansprüche

1. Katheterkopf für subkutane Verabreichung eines Wirkstoffs, der Katheterkopf wenigstens umfassend:
a) ein Kanülengehäuse (2) mit einer, in einem Gewebe zu plazierenden Kanüle (1), wobei in dem Kanülengehäuse (2) für den Wirkstoff ein Durchgangskanal (18, 19) zur Kanüle (1) ausgebildet ist und das Kanülengehäuse (2) eine auf das Gewebe flächig aufsetzbare und für eine Fixierung auf dem Gewebe vorbereitete Unterseite (12) aufweist,
b) einen Nadelhalter (3) mit einer Zuführung (5) für den Wirkstoff und einer in den Durchgangskanal (18, 19) des Kanülengehäuses (2) einzuführenden Verbindungsnadel (4), wobei der Nadelhalter (3) und das Kanülengehäuse (2) nach dem Einführen der Verbindungsnadel (4) sich lösbar aneinander befestigen oder befestigt werden, und
c) Führungsmittel (11), die zur Positionierung und bei dem Einführen der Verbindungsnadel (4) in den Durchgangskanal (18, 19) des Kanülengehäuses (2) den Nadelhalter (3) an dem Kanülengehäuse (2) führen,
**dadurch gekennzeichnet, daß**
d) der Nadelhalter (3) eine die Verbindungsnadel (4) axial umgebende Führungshülse (7) bildet, die
e) bei dem Einführen der Verbindungsnadel (4) eng gleitgeführt über einen zylindrischen Fortsatz (6) des Kanülengehäuses (2) geschoben wird, der einen Einlaß (9) und einen sich daran anschließenden Bereich des Durchgangskanals (18, 19) des Kanülengehäuses (2) umschließt.

2. Katheterkopf nach Anspruch 1, **dadurch gekennzeichnet, daß** die Führungshülse (7) eine die Verbindungsnadel (4) umgebende, geschlossene Mantelfläche aufweist.

3. Katheterkopf nach Anspruch 1 oder 2, **dadurch gekennzeichnet, daß** das Kanülengehäuse (2) einen scheibenförmigen hinteren Bereich (11) und einen den Durchgangskanal (18, 19) enthaltenden verdickten vorderen Bereich (10) aufweist, in oder an dem die Kanüle (1) befestigt ist und von dem der zylindrische Fortsatz (6) in den scheibenförmigen hinteren Bereich (11) abragt.

4. Katheterkopf nach dem vorhergehenden Anspruch, **dadurch gekennzeichnet, daß** zwischen dem zylindrischen Fortsatz (6) und einer Oberseite (13) des scheibenförmigen hinteren Bereichs (11) ein lichter Abstand verbleibt zum Überschieben der Führungshülse (7).

5. Katheterkopf nach wenigstens einem der vorhergehenden Ansprüche, **dadurch gekennzeichnet, daß** eine Oberseite (13) des Kanülengehäuses (2) für den Nadelhalter (3) eine Auflage und zusätzliche Gleitführung bei dem Einführen der Verbindungsnadel (4) bildet.

6. Katheterkopf nach dem vorhergehenden Anspruch, **dadurch gekennzeichnet, daß** sich die Oberseite (13) einer quer zur Verbindungsnadel (4) gewölbten Unterseite (14) des Nadelhalters (3) anschmiegt und dadurch die zusätzliche Gleitführung bildet; vorzugsweise erstreckt sich die zusätzliche Gleitführung parallel zum zylindrischen Fortsatz (6).

7. Katheterkopf nach wenigstens einem der vorhergehenden Ansprüche, **dadurch gekennzeichnet, daß** eine Unterseite (14) des Nadelhalters (3) bei dem Positionieren und Einführen der Verbindungsnadel (4) flächig auf einer Oberseite (13) eines hinteren Bereichs (11) des Kanülengehäuses (2) aufliegt.

8. Katheterkopf nach wenigstens einem der vorhergehenden Ansprüche, **dadurch gekennzeichnet, daß** der Nadelhalter (4) eine zu seiner Unterseite (14) symmetrische Oberseite aufweist, wobei die Unterseite (14) und die Oberseite sich vorzugsweise voneinander weg nach außen wölben.

9. Katheterkopf nach wenigstens einem der vorhergehenden Ansprüche, **dadurch gekennzeichnet, daß** eine Einstichnadel (N) für die Kanüle (1) das Kanülengehäuse (3) winkelig zu der Längsrichtung der eingeführten Verbindungsnadel (4) durchragt, wobei der Durchgangskanal (18, 19) für die Verbindungsnadel (4) winkelig in einen zusätzlichen Durchgang (20) für die Einstichnadel (N) einmündet.

10. Katheterkopf nach wenigstens einem der vorhergehenden Ansprüche, **dadurch gekennzeichnet, daß** die vorgefertigte Kanüle (1) bei einem Spritzgießen des Kanülengehäuses (2) in das Kanülengehäuse (2) eingespritzt wird.

## Claims

1. A catheter head for subcutaneous administration of an active substance, comprising at least the following:
a) a cannula housing (2) comprising a cannula (1) to be placed in a tissue, wherein a passage channel (18, 19) to said cannula (1) is formed in said cannula housing (2) for the active substance and said cannula housing (2) comprises an underside (12) which can be mould-positioned onto the issue and is prepared for fixing on the tissue;
b) a needle holder (3) including a feed (5) for the active substance and a connecting needle (4) to be inserted into said passage channel (18, 19) of said cannula housing (2), wherein said needle holder (3) and said cannula housing (2) detachably fix or are fixed to each other, once said connecting needle (4) has been inserted; and
c) guide means (11) for positioning said connecting needle which, when said connecting needle (4) is inserted into said passage channel (18, 19) of said cannula housing (2), guide said needle holder (3) on said cannula housing (2);
**characterized in that:**
d) said needle holder (3) forms a guide sleeve (7) which axially surrounds said connecting needle (4);
e) said guide sleeve (7) being narrowly slide-guided, when inserting said connecting needle (1), over a cylindrical extension (6) of said cannula housing (2), said extension (6) sheathing both an inlet (9) and a section of said passage channel (18, 19) of said cannula housing (2) following said inlet (9).

2. A catheter head as set forth in claim 1, **characterized in that** said guide sleeve (7) comprises a closed jacket surface surrounding said connecting needle (4).

3. A catheter head as set forth in claim 1 or 2, **characterized in that** said cannula housing (2) comprises a disc-shaped rear section (11) and a thicker front section (10) comprising said passage channel (18, 19), in or to which said cannula (1) is fixed, and from which said cylindrical extension (6) projects into said disc-shaped rear section (11).

4. A catheter head as set forth in the preceding claim, **characterized in that** a clearance remains between said cylindrical extension (6) and an upper side (13) of said disc-shaped rear section (11), for sliding the said guide sleeve (7) over.

5. A catheter head as set forth in at least one of the preceding claims, **characterized in that** an upper side (13) of the cannula housing (2) forms a support and an additional slideway for said needle holder (3) as the connecting needle (4) is inserted.

6. A catheter head as set forth in the preceding claim, **characterized in that** said upper side (13) is adapted to an underside (14) of said needle holder (3) which is curved in a transverse direction to said connecting needle (4), said upper side (13) thus forming said additional slideway; said additional slideway preferably extends parallel to said cylindrical extension (6).

7. A catheter head as set forth in at least one of the preceding claims, **characterized in that** an underside (14) of said needle holder (3) is mould-positioned on an upper side (13) of a rear section (11) of said cannula housing (2), when positioning and inserting said connecting needle (4).

8. A catheter head as set forth in at least one of the preceding claims, **characterized in that** said needle holder (3) comprises an upper side which is symmetrical to its underside (14), wherein said underside (14) and said upper side are preferably curve outwards away from each other.

9. A catheter head as set forth in at least one of the preceding claims, **characterized in that** a piercing needle (N) for said cannula (1) projects through said cannula housing (2) at an angle to the longitudinal direction of said inserted connecting needle (4), wherein said passage channel (18, 19) for said connecting needle (4) leads, at an angle, into an additional passage (20) for said piercing needle (N).

10. A catheter head as set forth in at least one of the preceding claims, **characterized in that** the prefabricated cannula (1) is formed as an integral part of said cannula housing (2) when injection-moulding said cannula housing (2).

## Revendications

1. Tête de cathéter pour administrer une substance active de manière sous-cutanée, ladite tête de cathéter comprenant au moins :
a) un boîtier à canule (2) comportant une canule (1) à placer dans un tissu, un canal traversant (18, 19) vers la canule étant ménagé dans le boîtier à canule (2) pour la substance active et le boîtier à canule (2) présentant une face inférieure (12) susceptible d'être posée à plat sur le tissu et préparée pour une fixation sur le tissu,
b) un porte-aiguille (3) présentant une conduite d'amenée (5) pour la substance active et une aiguille de jonction (4) à introduire dans le canal traversant (18, 19) du boîtier à canule (2), le porte-aiguille (3) et le boîtier à canule (2) se fixant ou étant fixés de façon détachable l'un sur l'autre après introduction de l'aiguille de jonction (4), et
c) des moyens de guidage (11) qui guident le porte-aiguille (3) sur le boîtier à canule (2) pour le positionnement et lors de l'introduction de l'aiguille de jonction (4) dans le canal traversant (18, 19) du boîtier à canule (2),
**caractérisée en ce que**
d) le porte-aiguille (3) forme une douille de guidage (7) entourant axialement l'aiguille de jonction (4), douille qui
e) lors de l'introduction de l'aiguille de jonction (4), est enfilée en étant guidée étroitement en coulissement par-dessus un prolongement cylindrique (6) du boîtier à canule (2), prolongement qui enferme une entrée (9) et une zone qui s'y raccorde du canal traversant (18, 19) du boîtier à canule (2).

2. Tête de cathéter selon la revendication 1, **caractérisée en ce que** la douille de guidage (7) présente une surface enveloppe fermée entourant l'aiguille de jonction (4).

3. Tête de cathéter selon l'une ou l'autre des revendications 1 et 2, **caractérisée en ce que** le boîtier à canule (2) comprend une zone arrière (11) en forme de disque et une zone avant épaissie (10) contenant le canal traversant (18, 19), dans ou sur laquelle est fixée la canule (1) et de laquelle fait saillie le prolongement cylindrique (6) jusque dans la zone arrière (11) en forme de disque.

4. Tête de cathéter selon la revendication précédente, **caractérisée en ce qu'**une distance libre subsiste entre le prolongement cylindrique (6) et une face supérieure (13) de la zone arrière (11) en forme de disque, qui sert à enfiler la douille de guidage (7).

5. Tête de cathéter selon l'une des revendications précédentes, **caractérisée en ce qu'**une face supérieure (13) du boîtier à canule (2) forme un support pour le porte-aiguille (3) et un guidage de coulissement supplémentaire lors de l'introduction de l'aiguille de jonction (4).

6. Tête de cathéter selon la revendication précédente, **caractérisée en ce que** la face supérieure (13) épouse une face inférieure (14) du porte-aiguille (3) bombée transversalement à l'aiguille de jonction (4) et forme ainsi le guidage de coulissement supplémentaire, le guidage de coulissement supplémentaire s'étendant de préférence parallèlement au prolongement cylindrique (6).

7. Tête de cathéter selon l'une des revendications précédentes, **caractérisée en ce qu'**une face inférieure (14) du porte-aiguille (3) repose à plat sur une face supérieure (13) de la zone arrière (11) du boîtier à canule (2) lors du positionnement et de l'introduction de l'aiguille de jonction (4).

8. Tête de cathéter selon l'une des revendications précédentes, **caractérisée en ce que** le porte-aiguille (3) présente une face supérieure symétrique par rapport à sa face inférieure (14), la face inférieure (14) et la face supérieure étant bombées de préférence vers l'extérieur en éloignement l'une de l'autre.

9. Tête de cathéter selon l'une des revendications précédentes, **caractérisée en ce qu'**une aiguille de percée (N) pour la canule (1) traverse le boîtier à canule (2) sous un angle par rapport à la direction longitudinale de l'aiguille de jonction (4) introduite, le canal traversant (18, 19) pour l'aiguille de jonction (4) débouchant sous un angle dans un passage supplémentaire (20) pour l'aiguille de percée (N).

10. Tête de cathéter selon l'une des revendications précédentes, **caractérisée en ce que** la canule préfabriquée (1) est injectée dans le boîtier à canule (2) lors d'une coulée par injection du boîtier à canule (2).
